# EUROPEAN PATENT APPLICATION

(11) **EP 4 741 381 A1**
(43) Date of publication of application: **13.05.2026**
(21) Application number: 24835989.5
(22) Date of filing: 28.06.2024
(51) Int. Cl.: C07D 285/08

(54) **NOVEL METHOD FOR PRODUCING 3-METHYL-1,2,4-THIADIAZOLE-5-CARBOHYDRAZIDE**

(30) Priority: 03.07.2023 JP 2023109328
(71) Applicant: AGC Inc., Chiyoda-ku Tokyo 100-8405 (JP)
(72) Inventor: OIKAWA, Daiki, Tokyo 100-8405 (JP); NAKAI, Katsuya, Tokyo 100-8405 (JP); SUZUKI, Hideo, Tokyo 100-8405 (JP)
(74) Representative: Müller-Boré & Partner Patentanwälte PartG mbB
(86) International application number: PCT/JP2024/023469
(87) International publication number: WO 2025/009476

(57) **Abstract**

The present invention aims to provide a method for producing 3-methyl-1,2,4-thiadiazole-5-carbohydrazide safely and in high yield. The present invention relates to a method for producing 3-methyl-1,2,4-thiadiazole-5-carbohydrazide or a salt thereof, via a compound represented by the formula (2): 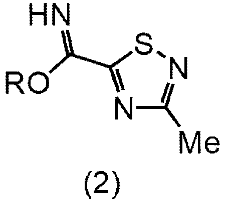 wherein R is a C₁₋₄ alkyl group, which is a novel compound, or a salt thereof. According to the present invention, a safe and high-yielding production method of 3-methyl-1,2,4-thiadiazole-5-carbohydrazide, which is an important synthetic intermediate for fezolinetant useful as a medicament for the treatment of sex hormone-dependent diseases, can be provided.

## Description

### [Technical Field]

The present invention relates to a novel, safe, and practical method for producing 3-methyl-1,2,4-thiadiazole-5-carbohydrazide which is an important synthetic intermediate for fezolinetant which was developed as a selective antagonist of the neurokinin (NK)-3 receptor, and is particularly useful as a compound for the treatment of sex hormone-dependent diseases.

### [Background Art]

Fezolinetant (generic name) (VEOZAH (registered trademark)) was developed as a selective antagonist of NK-3 receptor, and is particularly useful as a compound for the treatment and/or prophylaxis of sex hormone-dependent diseases (e.g., moderate to severe vasomotor symptoms associated with menopause, such as facial flushing, hot flashes, and the like). Fezolinetant is (R)-(4-fluorophenyl)-(8-methyl-3-(3-methyl-1,2,4-thiadiazol-5-yl)-5,6-dihydro-[1,2,4]triazolo[4,3-a]pyrazin-7(8H)-yl)methanone and described in Patent Literature 1.

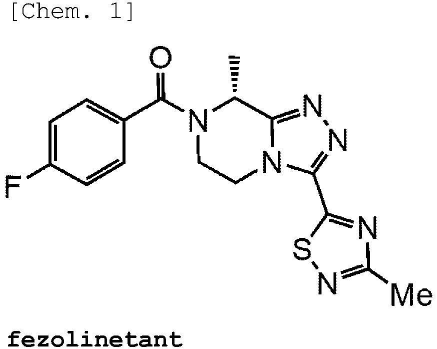

Deuterated fezolinetant, (R)-(4-fluorophenyl)-(8-methyl-3-(3-(methyl-d3)-1,2,4-thiadiazol-5-yl)-5,6-dihydro-[1,2,4]triazolo[4,3-a]pyrazin-7(8H)-yl)methanone was also developed for the same purpose and is described in Patent Literature 2.

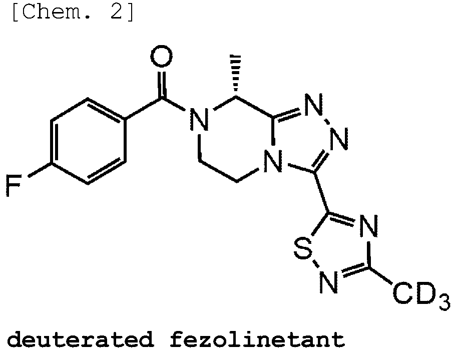

Patent Literatures 1 and 2 disclose, as a synthesis method of fezolinetant and deuterated fezolinetant, a synthesis method going through 3-methyl-1,2,4-thiadiazole-5-carbohydrazide or a deuterated form thereof, which is a compound represented by the formula (I): 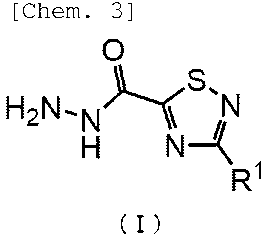 wherein R¹ is methyl or methyl-d₃, as an important intermediate.

Patent Literature 3 discloses a synthesis method of the non-deuterated intermediate, 3-methyl-1,2,4-thiadiazole-5-carbohydrazide (2n): 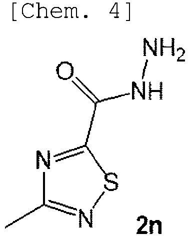 and in the synthesis method, it is prepared from the corresponding methyl ester (a compound represented by the following formula (2.2n)), which in turn is prepared in a single step from acetamide (a compound represented by the following formula (2.0n)), chlorocarbonylsulfenyl chloride (a compound represented by the following formula (2.0n')), and methyl cyanoformate (a compound represented by the following formula (2.0n")).

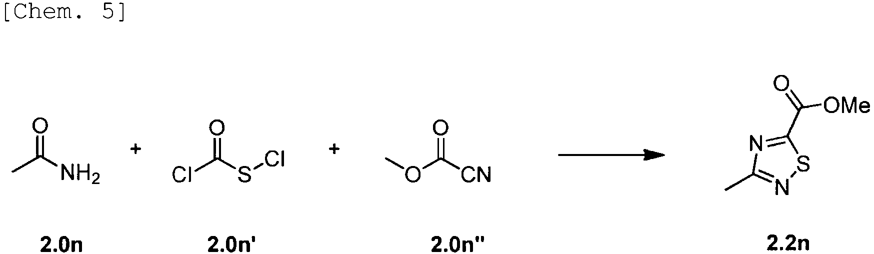

However, chlorocarbonylsulfenyl chloride and methyl cyanoformate are hazardous reagents that pose problems in large-scale procurement and are difficult to scale up. This synthetic route also results in sulfur impurities that adversely affect the quality of the final pharmaceutical product. Even after optimization, the overall yield of 3-methyl-1,2,4-thiadiazole-5-carbohydrazide remains below 30%. The synthesis of the corresponding deuterated intermediate, 3-(methyl-d₃)-1,2,4-thiadiazole-5-carbohydrazide, also has similar drawbacks (Patent Literature 2).

Patent Literature 4 discloses, as a synthesis method of an intermediate compound represented by the following formula (I-1): 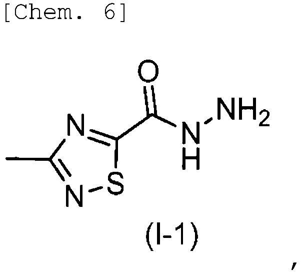
a synthesis method including alkoxycarbonylation by lithium exchange reaction or by carbon monoxide insertion reaction in the presence of a metal catalyst of the corresponding halogenated intermediate, or a compound represented by the following formula (III-1-a): 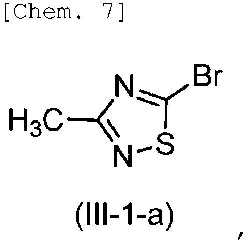
or a compound represented by the following formula (III-1-b): 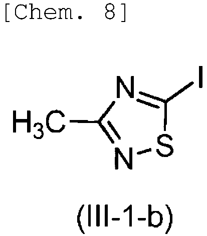
to obtain the corresponding ester form, a compound represented by the following formula (II-1-b): 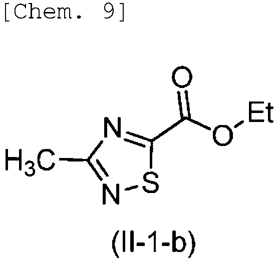
or a salt thereof, a synthesis method via which is disclosed. However, the yield of the alkoxycarbonylation reaction by lithium exchange is as low as 29%. Furthermore, although the alkoxycarbonylation reaction via carbon monoxide insertion in the presence of a metal catalyst affords a compound represented by formula (II-1-b) in good yield, it requires the use of toxic carbon monoxide gas and is limited in terms of scale-up, thus leaving issues for practical application.

Therefore, the development of a safe, high-yielding, scalable, and practical method for producing 3-methyl-1,2,4-thiadiazole-5-carbohydrazide which is an important synthetic intermediate for fezolinetant is desired.

### [Citation List]

### [Patent Literature]

[Patent Literature 1]
   WO 2014/154895
[Patent Literature 2]
   WO 2019/012033
[Patent Literature 3]
   WO 2013/050424
[Patent Literature 4]
   WO 2020/128003

### [Summary of Invention]

### [Technical Problem]

An object of the present invention is to provide a safe, high-yielding, and practical method for producing a compound represented by the following formula (I): 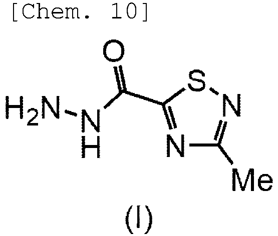 or a salt thereof which is an important synthetic intermediate for fezolinetant.

### [Solution to Problem]

The present inventors have conducted intensive studies in an attempt to solve the above-mentioned problem and found that a compound represented by the formula (3): 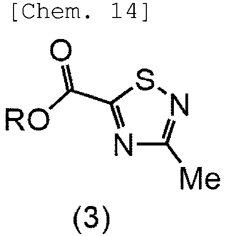
wherein R is a C₁₋₄ alkyl group,
or a salt thereof can be produced in a high yield without using carbon monoxide gas, by using a compound represented by the formula (2): 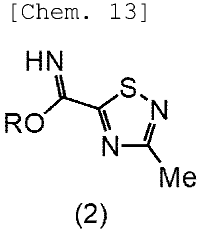
wherein R is as defined above,
or a salt thereof which is obtained by a step of reacting a compound represented by the formula (1): 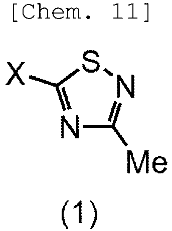
wherein X is a halogen atom,
or a salt thereof with an alkali metal cyanide in a solvent containing an aprotic polar solvent in the presence of an organic base, and then reacting the compound with MOR wherein M is an alkali metal and R is as defined above, in the presence of alcohol represented by the following formula:

   [Chem. 12] ROH
wherein R is as defined above,
and that, as a result, a safe and practical production method of a compound represented by the formula (I): 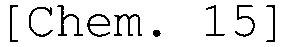 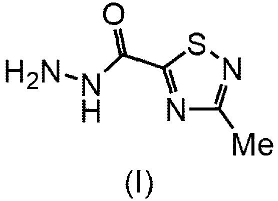
or a salt thereof, which is an important synthetic intermediate for fezolinetant, can be provided, which resulted in the completion of the present invention.

That is, the present invention is as follows.
[1] A method for producing a compound represented by the formula (2): 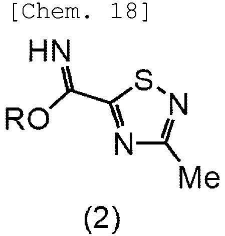
   wherein R is a C₁₋₄ alkyl group, or a salt thereof, comprising a step of reacting a compound represented by the formula (1): 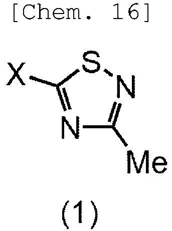
   wherein X is a halogen atom, or a salt thereof
   with an alkali metal cyanide in a solvent containing an aprotic polar solvent in the presence of an organic base, followed by adding an alcohol represented by the following formula:

      [Chem. 17] ROH
   wherein R is as defined above, and then reacting the compound with MOR wherein M and R are as defined above.
[2] The production method of the above-mentioned [1], wherein
   X is a chlorine atom or a bromine atom.
[3] The production method of the above-mentioned [1], further comprising additional addition of an organic base when adding the alcohol.
[4] The production method of the above-mentioned [1], wherein
   the organic base is triethylamine, N,N-diisopropylethylamine, N-methylmorpholine, pyridine, 2,6-dimethylpyridine, N,N-dimethyl-4-aminopyridine, imidazole, 1,8-diazabicyclo[5.4.0]undec-7-ene, 1,4-diazabicyclo[2.2.2]octane, or 1,5-diazabicyclo[4.3.0]non-5-ene.
[5] The production method of the above-mentioned [1], wherein
   the alkali metal cyanide is potassium cyanide or sodium cyanide.
[6] The production method of the above-mentioned [1], wherein
   the alcohol is methanol or ethanol.
[7] The production method of the above-mentioned [1], wherein
   the aprotic polar solvent is N,N-dimethylformamide, N,N-dimethylacetamide, N-methyl-2-pyrrolidone, or dimethyl sulfoxide.
[8] The production method of the above-mentioned [1], wherein
   the solvent further comprises a non-polar solvent.
[9] The production method of the above-mentioned [8], wherein
   the non-polar solvent is toluene, n-hexane, cyclohexane, or n-heptane.
[10] A method for producing a compound represented by the formula (3): 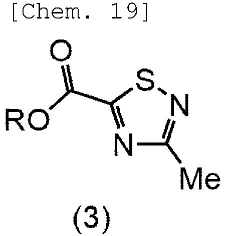 wherein R is as defined above, or a salt thereof,
   comprising a step of acid hydrolyzing a compound represented by the formula (2) obtained by the production method of any of the above-mentioned [1] to [9], or a salt thereof.
[11] The production method of the above-mentioned [10], wherein
   the acid is sulfuric acid, hydrochloric acid, hydrobromic acid, nitric acid, phosphoric acid, trifluoroacetic acid, methanesulfonic acid, p-toluenesulfonic acid, aluminum chloride, boron trifluoride, titanium chloride, or zinc chloride.
[12] A method for producing a compound represented by the formula (I): 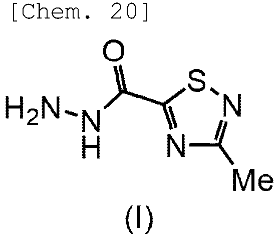 or a salt thereof,
   comprising a step of reacting the compound represented by the formula (3) which is obtained by the production method of the above-mentioned [10] or a salt thereof with hydrazine or a hydrate thereof in a solvent.
[13] A compound represented by the formula (2): 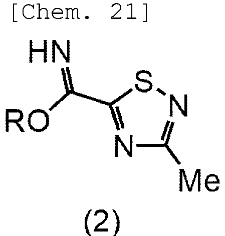 wherein R is a C₁₋₄ alkyl group, or a salt thereof.

### [Advantageous Effects of Invention]

According to the present invention, 3-methyl-1,2,4-thiadiazole-5-carbohydrazide represented by the aforementioned formula (I) or a salt thereof, which is an important synthetic intermediate for fezolinetant, can be produced in good yield without using toxic carbon monoxide gas, but by involving 3-methyl-1,2,4-thiadiazole-5-carboimidic acid ester represented by the aforementioned formula (2) which is a novel compound. Therefore, a practical production method of fezolinetant can be provided which is safer than conventional methods and can be scaled up.

### [Description of Embodiments]

The definitions of the terms used in the present specification are described below.

In the present specification, a compound represented by a formula may be referred to by adding the formula number to the "compound". For example, a compound represented by the formula (1) is referred to as "compound (1)".

In the present specification, a numerical range shown by "~" or "-" means a numerical range with the numbers before and after "~" or "-" as the lower limit or upper limit.

In the present specification, when the element symbol "C" is used with a numerical range indicated by numbers before and after "-" attached to the name of any group, it represents any group having an integer number of carbon atoms, with the numbers before and after "-" as the lower limit and upper limit. For example, an alkyl group having 1 to 4 carbon atoms is sometimes denoted as a "C₁₋₄ alkyl group" which represents each of -CH₃, -C₂H₅, -C₃H₇, -C₄H₉, and the like.

In the present specification, the "halogen atom" means a fluorine atom, a chlorine atom, a bromine atom, or an iodine atom.

In the present specification, the "C₁₋₄ alkyl group" means a linear or branched chain saturated hydrocarbon group having 1 to 4 carbon atoms, and examples of the C₁₋₄ alkyl group include methyl, ethyl, propyl, isopropyl, butyl, isobutyl, sec-butyl, and tert-butyl.

In the present specification, the "solvent containing an aprotic polar solvent" means that the solvent contains an aprotic polar solvent. The "solvent containing an aprotic polar solvent" is not particularly limited, and examples thereof include a solvent consisting of at least one aprotic polar solvent, a mixed solvent of an aprotic polar solvent and a non-polar solvent, and the like.

In the present specification, the "aprotic polar solvent" is not particularly limited, and examples thereof include amide solvents such as N,N-dimethylformamide, N,N-dimethylacetamide, N-methyl-2-pyrrolidone, and the like; sulfoxide solvents such as dimethyl sulfoxide and the like; nitrile solvents such as acetonitrile, propionitrile, and the like; and the like. Among these, N,N-dimethylformamide, N,N-dimethylacetamide, N-methyl-2-pyrrolidone, or dimethyl sulfoxide is preferred.

In the present specification, the "non-polar solvent" is not particularly limited, and examples thereof include aromatic hydrocarbon solvents such as toluene, xylene, and the like; aliphatic hydrocarbon solvents such as n-hexane, cyclohexane, n-heptane, and the like; and the like. Among these, toluene, n-hexane, cyclohexane, or n-heptane is preferred.

In the present specification, examples of the "alkali metal cyanide" include lithium cyanide, potassium cyanide, sodium cyanide, cesium cyanide, and the like.

In the present specification, the "organic base" is an organic compound that acts as a base. The organic base is generally a proton acceptor containing a nitrogen atom that can be easily protonated, and examples thereof include amine, nitrogen-containing heterocyclic compounds, and the like. The "organic base" is not particularly limited and examples thereof include triethylamine (TEA), N,N-diisopropylethylamine (DIPEA), N-methylmorpholine (NMO), N,N-dimethyl-4-aminopyridine (DMAP), imidazole, 1,8-diazabicyclo[5.4.0]undec-7-ene (DBU), 1,4-diazabicyclo[2.2.2]octane (DABCO), 1,5-diazabicyclo[4.3.0]non-5-ene (DBN), and the like.

In the present specification, the "prophylaxis" includes preventing the onset of a disease, delaying the onset of a disease, and preventing the development of a pathology in a subject.

In the present specification, the "treatment" includes curing a disease, ameliorating the pathology of a disease (e.g., one or more symptoms), and inhibiting the progression (of the severity) of a disease in a subject.

In the present specification, the "subject" refers to a subject to which a medicament (pharmaceutical composition) containing an effective amount of an active ingredient necessary for the prophylaxis and/or treatment of a disease or a pathological condition of a disease is administered. The "subject" may be a human or a non-human animal, particularly a mammal (e.g., mouse, rat, guinea pig, hamster, rabbit, cat, dog, cow, sheep, monkey, etc.).

In the present specification, "a salt thereof" is not particularly limited as long as it is a salt formed by reacting the target compound with a base or an acid. Since the present invention relates to a method for producing an important synthetic intermediate for fezolinetant, which is useful as a medicament, a salt that can be used as a medicament (a pharmaceutically acceptable salt) is preferred. When each compound used in the production method of the present invention forms a basic salt or an acidic salt by reacting with a base or an acid, it refers to such salt.

Examples of the "basic salt" include sodium salt, potassium salt, lithium salt, magnesium salt, calcium salt, aluminum salt, ammonium salt, tetramethylammonium salt, N-methylmorpholine salt, diethylamine salt, triethylamine salt, tributylamine salt, diisopropylethylamine salt, dicyclohexylamine salt, N-methylpiperidine salt, pyridine salt, 4-pyrrolidinopyridine salt, picoline salt, choline salt, diolamine salt, meglumine salt, olamine salt, tromethamine salt, 2-(diethylamino)ethanol salt, 4-(2-hydroxyethyl)morpholine salt, arginine salt, glycine salt, lysine salt, benzathine salt and the like.

Examples of the "acidic salt" include hydrofluoride, hydrochloride, hydrobromide, hydroiodide, nitrate, perchlorate, sulfate, bisulfate, borate, camsylate, cyclamate, carbonate, bicarbonate, phosphate, hexafluorophosphate, formate, lactate, trifluoroacetate, mesylate, trifluoromethanesulfonate, esilate, edisilate, benzoate, besylate, tosylate, acetate, malate, fumarate, succinate, malonate, citrate, ascorbate, tartrate, oxalate, maleate, adipate, nicotinate, aspartate, gluconate, glucuronate, pyroglutamate, stearate, palmitate, tannate, isethionate, naphthoate, orotate, pamoate, xinafoate, gluceptate, and hibenzate.

In the present invention, a compound represented by the formula (I): 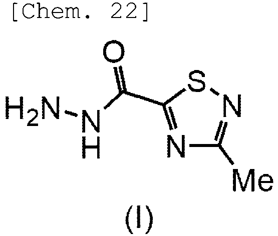 (compound (I)) or a salt thereof also includes a compound in which the 3-position methyl group is deuterated, i.e., 3-(methyl-d3)-1,2,4-thiadiazole-5-carbohydrazide or a salt thereof.

### (Production method of the present invention)

The production method of the present invention is described in the following.

A compound represented by the formula (I): 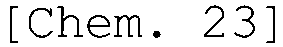 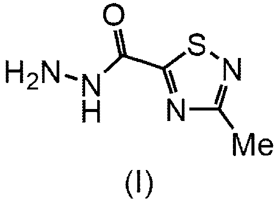 (compound (I)) or a salt thereof can be produced by the following production method, the below-mentioned Example, a method analogous thereto, and the like.

Each raw material compound may form a salt as long as it does not inhibit the reaction, and examples of such salts include the same salts as those described above.

Unless a specific production method is described, the starting compounds can be easily obtained from those on the market and used, or can be produced by a method known per se or a method analogous thereto. In addition, intermediates produced in the following production methods may be isolated and purified by a method such as column chromatography, recrystallization, or distillation, or may be used in the next step without isolation.

In the present specification, the contents of all patent literature, non patent literature, or references expressly cited are hereby incorporated by reference in their entirety.

In the present invention, compound (I) or a salt thereof is produced by a step of reacting a compound represented by the formula (1): 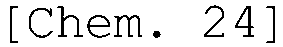 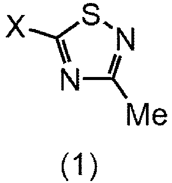
wherein X is a halogen atom (compound (1)) or a salt thereof and an alkali metal cyanide in a solvent containing an aprotic polar solvent in the presence of an organic base, and then reacting the compound with MOR wherein M is an alkali metal and R is as defined above, by adding an alcohol represented by the following formula:

   [Chem. 25] ROH
wherein R is a C₁₋₄ alkyl group, to produce a compound represented by the formula (2): 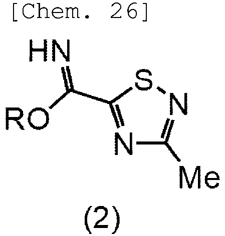
wherein R is as defined above (compound (2)) or a salt thereof (step 1), then a step of producing a compound represented by the formula (3): 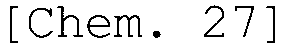 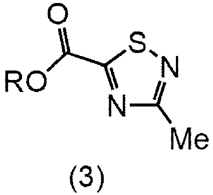
wherein R is as defined above (compound (3)) or a salt thereof by acid hydrolysis of compound (2) or a salt thereof (step 2), and reacting compound (3) or a salt thereof with a hydrazine or a hydrate thereof (step 3).

Each step in the production method of the present invention is described in detail in the following.

### (Step 1)

In this step, compound (1) or a salt thereof is reacted with an alkali metal cyanide (M'CN) in a solvent containing an aprotic polar solvent in the presence of an organic base (step 1-1), an alcohol represented by the following formula:

[Chem. 28] ROH

wherein R is a C₁₋₄ alkyl group, is added, and the compound is reacted with MOR wherein M is an alkali metal (preferably, sodium or potassium, more preferably sodium) and R is as defined above (step 1-2) to produce compound (2) or a salt thereof. The reaction in this step proceeds via 5-cyano-3-methyl-1,2,4-thiadiazole, but there is no need to isolate or purify 5-cyano-3-methyl-1,2,4-thiadiazole, and compound (2) or a salt thereof can be obtained in one pot. 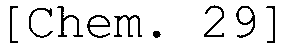 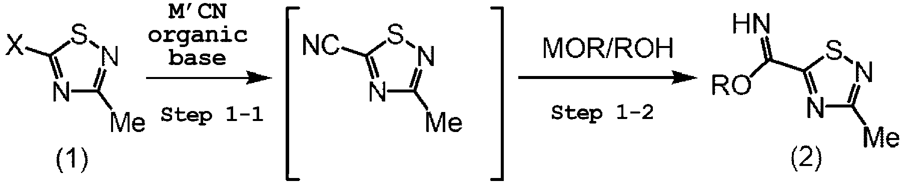
wherein M' is an alkali metal, and other symbols are as defined above.

Compound (1) or a salt thereof, which is synthesized by a method known per se (see, for example, WO 2020/128003 (Patent Literature 4), Chem. Ber., 1957, 90, 182-187, etc.) or a method analogous thereto, can be suitably used. Compound (1) or a salt thereof is preferably 5-chloro-3-methyl-1,2,4-thiadiazole or 5-bromo-3-methyl-1,2,4-thiadiazole, more preferably 5-chloro-3-methyl-1,2,4-thiadiazole.

Alkali metal cyanide (M'CN) to be used is not particularly limited, and examples thereof include lithium cyanide, potassium cyanide, sodium cyanide, cesium cyanide, and the like, and potassium cyanide or sodium cyanide is preferred, and sodium cyanide is more preferred.

The amount of the alkali metal cyanide to be used is 0.7 to 3 moles, preferably 0.8 to 1.5 moles, per 1 mole of compound (1) or a salt thereof.

The organic base to be used is not particularly limited and examples thereof include triethylamine (TEA), N,N-diisopropylethylamine (DIPEA), N-methylmorpholine (NMO), pyridine, 2,6-dimethylpyridine, N,N-dimethyl-4-aminopyridine (DMAP), imidazole, 1,8-diazabicyclo[5.4.0]undec-7-ene (DBU), 1,4-diazabicyclo[2.2.2]octane (DABCO), 1,5-diazabicyclo[4.3.0]non-5-ene (DBN), and the like, and N,N-diisopropylethylamine (DIPEA), N,N-dimethyl-4-aminopyridine (DMAP), 1,8-diazabicyclo[5.4.0]undec-7-ene (DBU), or 1,4-diazabicyclo[2.2.2]octane (DABCO) is preferred, and 1,4-diazabicyclo[2.2.2]octane (DABCO) is more preferred.

The amount of the organic base to be used is generally 0.05 to 2 moles, preferably 0.05 to 0.2 moles, per 1 mole of compound (1) or a salt thereof.

This reaction can be performed in a solvent containing an aprotic polar solvent.

Examples of solvent containing an aprotic polar solvent include the solvents described above. A solvent consisting of a single aprotic polar solvent, or a mixed solvent of an aprotic polar solvent and a non-polar solvent is preferred, and a mixed solvent of an aprotic polar solvent and a non-polar solvent is more preferred.

The aprotic polar solvent constituting the mixed solvent of the aprotic polar solvent and the non-polar solvent is not particularly limited, and examples thereof include amide solvents such as N,N-dimethylformamide, N,N-dimethylacetamide, N-methyl-2-pyrrolidone, and the like; sulfoxide solvents such as dimethyl sulfoxide and the like; nitrile solvents such as acetonitrile, propionitrile, and the like; and the like. Among these, N,N-dimethylformamide, N,N-dimethylacetamide, N-methyl-2-pyrrolidone, or dimethyl sulfoxide is preferred, and N,N-dimethylformamide is more preferred.

The non-polar solvent constituting the mixed solvent of the aprotic polar solvent and the non-polar solvent is not particularly limited, and examples thereof include aromatic hydrocarbon solvents such as toluene, xylene and the like; aliphatic hydrocarbon solvents such as n-hexane, cyclohexane, n-heptane and the like; and the like. Among these, toluene, n-hexane, cyclohexane, or n-heptane is preferred, and toluene is more preferred.

The mixing ratio (v/v) of the mixed solvent of the aprotic polar solvent and the non-polar solvent is not particularly limited, but aprotic polar solvent/non-polar solvent is generally in the range of 0.1 to 100, preferably 0.2 to 5, more preferably 0.5 to 3.

The alkali metal alkoxide (MOR) used in step 1-2 is not particularly limited as long as it is an alkali metal alkoxide having 1 to 4 carbon atoms, and sodium methoxide, potassium methoxide, sodium ethoxide, or potassium ethoxide is preferred, and sodium methoxide or sodium ethoxide is more preferred. The amount of the alkali metal alkoxide to be used is generally 0.05 to 2 moles, preferably 0.08 to 0.2 moles, per 1 mole of compound (1) or a salt thereof. The alcohol used in step 1-2 is an alcohol capable of forming an alkoxide of the alkali metal alkoxide (specifically, for example, sodium ethoxide/ethanol).

Step 1-2 can also be performed in the presence of an organic base as necessary. Examples of the organic base include those described above, and triethylamine (TEA) is preferred.

Adding an organic base has the advantage of inactivating any alkali metal cyanide (M'CN) remaining in the reaction system during work-up and suppressing the generation of hydrogen cyanide, thereby allowing this step to be performed safely.

The amount of the organic base to be used is generally 0.05 to 2 moles, preferably 0.1 to 0.3 moles, per 1 mole of compound (1) or a salt thereof.

The reaction temperature of step 1-1 is generally 0°C to 120°C, and the reaction time is generally 3 to 24 hr.

The reaction temperature of step 1-2 is generally -10°C to 20°C, and the reaction time is generally 0.5 to 4 hr.

### (Step 2)

In this step, compound (2) or a salt thereof obtained in the aforementioned step 1 is acid hydrolyzed to produce compound (3) or a salt thereof.

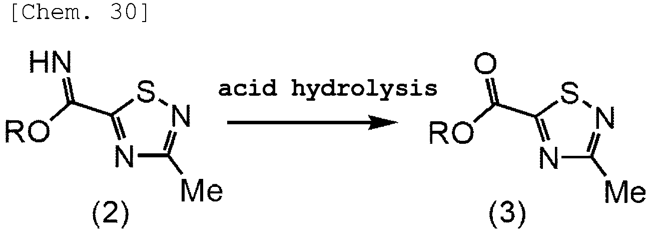

The acid to be used may be either a protonic acid or a Lewis acid, and the protonic acid is preferably sulfuric acid, hydrochloric acid, hydrobromic acid, nitric acid, phosphoric acid, trifluoroacetic acid, methanesulfonic acid, or p-toluenesulfonic acid, more preferably sulfuric acid or hydrochloric acid, and the Lewis acid is preferably aluminum chloride (AlCl₃), boron trifluoride (BF₃), titanium chloride (TiCl₃), or zinc chloride (ZnCl₂). The acid may be diluted with water before use.

The amount of the acid to be used is generally 1 to 3 moles, preferably 1.03 to 1.5 moles, per 1 mole of compound (2) or a salt thereof.

The reaction temperature is generally 0°C to 20°C, preferably 0°C to 10°C, and the reaction time is generally 0.5 to 4 hr.

### (Step 3)

In this step, compound (3) or a salt thereof obtained in the aforementioned step 2 is reacted with hydrazine or a hydrate thereof in a solvent to produce compound (I) or a salt thereof.

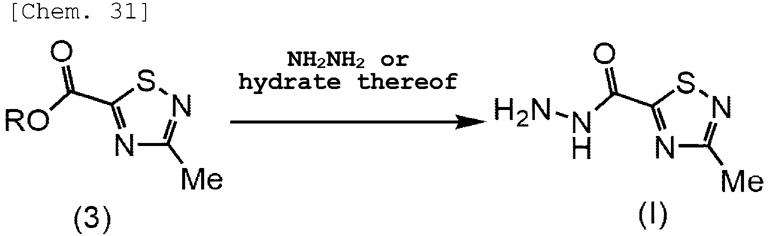

The hydrazine or a hydrate thereof is not particularly limited and hydrazine monohydrate is preferred.

The amount of the hydrazine or a hydrate thereof to be used is generally 1 to 2 moles, preferably 1 to 1.2 mole, per 1 mole of compound (3) or a salt thereof.

This reaction can be performed in a solvent that does not affect the reaction. The reaction solvent is not particularly limited, and examples thereof include aromatic hydrocarbons such as toluene, xylene, and the like; amides such as N,N-dimethylformamide, N,N-dimethylacetamide, and the like; alcohols such as methanol, ethanol, isopropanol, and the like; nitriles such as acetonitrile and the like, or a mixture thereof. Among these, a mixed solvent of alcohol and aromatic hydrocarbon is preferred, and a mixed solvent of isopropanol-toluene is more preferred.

The reaction temperature is generally -10°C to 30°C, preferably 0°C to 10°C, and the reaction time is generally 3 to 24 hr.

After completion of the reaction, compound (I) or a salt thereof can be isolated and/or purified from the reaction mixture according to a conventional method using a separation means such as concentration, crystallization, recrystallization, distillation, solvent extraction, fractionation, chromatography and the like.

Compound (I) or a salt thereof obtained in the aforementioned step 3 can be converted to fezolinetant by a method known per se (see, for example, WO 2014/154895 (Patent Literature 1), WO 2019/012033 (Patent Literature 2)), or a method analogous thereto.

The production method of the present invention does not use any carbon monoxide gas, the use of which on an industrial scale is restricted, and therefore has the advantage of being able to synthesize compound (I) or a salt thereof in good yield by a safe and simple operation compared to conventional methods.

### (Compound of the present invention)

The production method of the present invention is characterized by not using carbon monoxide, and by using a compound represented by the formula (2): 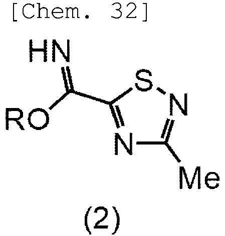 wherein R is as defined above (compound (2)) or a salt thereof as a synthetic intermediate, it can produce compound (I) or a salt thereof, which is an important synthetic intermediate for fezolinetant, more safely and in a higher yield than conventional methods.

Compound (2) or a salt thereof is a novel compound and useful as a novel intermediate compound in the synthesis of fezolinetant.

### [Example]

The present invention is specifically explained in detail in the following by referring to Reference Examples and Examples; however, the present invention is not limited to these Examples.

In the present specification, % means mol/mol% for yield and wt% for others unless particularly indicated. The room temperature refers to a temperature of from 15 to 30°C unless particularly indicated.

Nuclear magnetic resonance spectra (NMR) were measured using ECZ400S FT-NMR manufactured by JEOL Ltd. ¹H-NMR was measured at 400 MHz using tetramethylsilane as the reference. When a deuterated solvent was used as the measurement solvent, the name of the deuterated solvent is indicated.

Infrared absorption spectrum (IR) was measured by the ATR method using Nic-plan/Nicolet 6700 manufactured by Thermo Fisher Scientific.

Liquid chromatography-mass spectrometry (LC-MS) was performed using UltiMate 3000 and Q Exactive manufactured by Thermo Fisher Scientific.

High-performance liquid chromatograph (HPLC) used was 1220 Infinity LC manufactured by Agilent Technologies, and the column used was Atlantis T3 (3 µm (particle size), 4.6×150 mm, manufactured by Waters).

In addition, the abbreviations used in the following Examples have the following meanings.

s: singlet
t: triplet
q: quartet
br: broad
J: coupling constant
CDCl₃: deuterochloroform
DMSO-d₆: deuterodimethyl sulfoxide
CH₂Cl₂: dichloromethane
NaOH: sodium hydroxide
NaCN: sodium cyanide
DABCO: 1,4-diazabicyclo[2.2.2]octane
DMF: N,N-dimethylformamide
TEA: triethylamine
NaOEt: sodium ethoxide
EtOH: ethanol
iPrOH: isopropanol

### [Reference Example 1]

### Production of 5-chloro-3-methyl-1,2,4-thiadiazole (1-1)

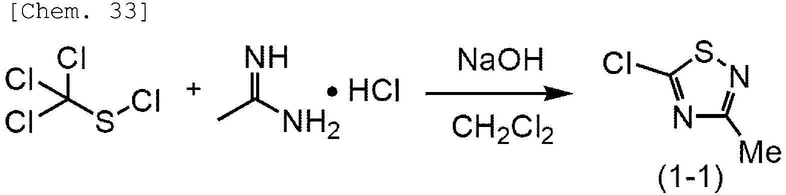

Under a nitrogen atmosphere (normal pressure) at 0°C, to acetamidine hydrochloride (100 g, 1.06 mol), trichloromethanesulfenyl chloride (196.8 g, 1.06 mol), and 6 M aqueous sodium hydroxide solution (902.7 g, 4.4 eq.) was added dichloromethane (530 mL (5.3 v/w)), and the reaction mixture was stirred at 25°C for 2 hr. Thereafter, the reaction mixture was partitioned, and the obtained organic layer was washed successively with 6 M hydrochloric acid (350 mL (3.5 v/w)) and water (350 g (3.5 v/w)). The obtained organic layer was subjected to simple distillation (vacuum pressure: 400-15 Torr, internal temperature: 25 to 60°C) to obtain the title compound (1-1) in 54% yield (quantitative by LC).
¹H-NMR (400 MHz, CDCl₃) δ 2.64 (s,3H).

### [Example 1]

### Production of ethyl 3-methyl-1,2,4-thiadiazole-5-carboimidate (2-1)

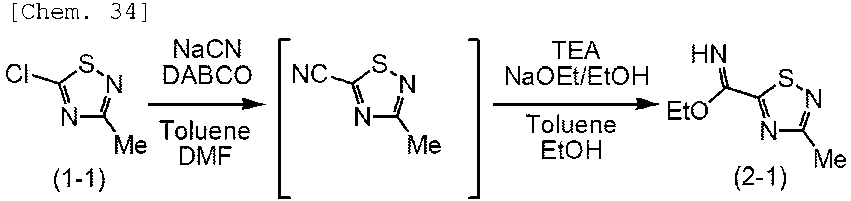

Under a nitrogen atmosphere (normal pressure) at 15°C, compound (1-1) (4 g, 29.72 mmol) obtained in Reference Example 1, DABCO (0.667 g, 5.94 mmol), toluene (3.46 mL), DMF (3.79 mL), and sodium cyanide (1.63 g, 32.7 mmol) were mixed, and the reaction mixture was stirred at the same temperature for 4 hr. Thereafter, at 15°C, to the reaction mixture were added toluene (12.16 mL), triethylamine (0.60 g, 5.94 mmol), ethanol (1.36 g, 29.72 mmol), and 20% sodium ethoxide/ethanol solution (0.83 mL, 2.43 mmol), and the mixture was further stirred at the same temperature for 1 hr. To the reaction mixture was added water, a partitioning operation was performed twice, and the organic layer was washed with 10% aqueous sodium chloride solution to obtain a toluene solution of the title compound (2-1) in 67% yield (quantitative by LC).
¹H-NMR (400 MHz, DMSO-d₆) δ 0.52 (t,J=7.3Hz,3H), 1.85 (s,3H), 3.54 (q,J=7.3Hz,2H), 8.49 (s,1H);
IR (cm⁻¹) 3286,2900-3000,1653,1450;
LC-MS m/z 172.0535([M+H]⁺)

The hydrogen cyanide gas concentration in the gas phase of the reaction vessel during this reaction was below the detection limit (≤10 ppm), and it could be confirmed that the reaction was safe.

### [Example 2]

### Production of ethyl 3-methyl-1,2,4-thiadiazole-5-carboxylate (3-1)

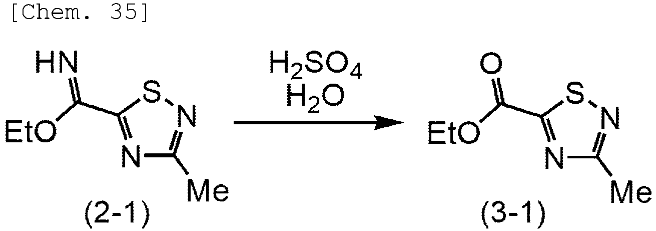

Under a nitrogen atmosphere, a toluene solution of compound (2-1) (concentration 14%) (2201.3 g, 1.82 mol) obtained in Example 1 was cooled to 5°C, water and concentrated sulfuric acid (347.2 g (1.9 eq.)) were added dropwise, and the mixture was stirred at 5°C for 1 hr. Thereafter, the reaction mixture was partitioned. To the obtained organic phase was added 10% aqueous sodium carbonate solution until the pH reached 5 to 6, and the mixture was partitioned. The obtained organic layer was quantified, and a toluene solution of the title compound (3-1) was obtained in 98% yield (quantitative by LC).
¹H-NMR (400 MHz, DMSO-d₆) δ 1.28 (t,J=8.0Hz,3H), 2.59 (s,3H), 4.35 (q,J=7.6Hz,2H).

### [Example 3]

### Production of 3-methyl-1,2,4-thiadiazole-5-carbohydrazide (I)

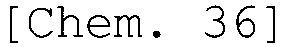

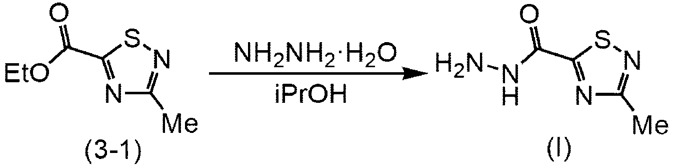

Under a nitrogen atmosphere, hydrazine monohydrate (93.7 g, 1.85 mol) was added to isopropanol (1519 mL (5v/w)), and the mixture was cooled to 0 to 10°C. A toluene solution of compound (3-1) (concentration 15%) (2052.6 g, 1.76 mol) obtained in Example 2 was added dropwise thereto, and the mixture was stirred at 0 to 10°C for 12 hr. Thereafter, the reaction mixture was filtered under reduced pressure and the residue was washed with isopropanol (400 mL (4 v/w)) and dried under reduced pressure to obtain the title compound (I) in 94% yield (quantitative by LC).
¹H-NMR (400 MHz, DMSO-d₆) δ 2.56 (s,3H), 4.82 (br,2H), 10.56 (br,1H).

### [Example 4]

### Production of methyl 3-methyl-1,2,4-thiadiazole-5-carboimidate (2-2)

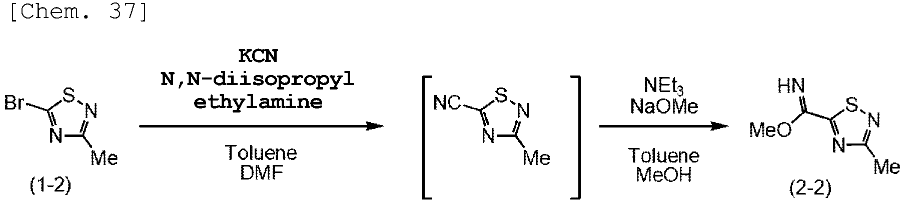

Under a nitrogen atmosphere (normal pressure) at 15°C, 5-bromo-3-methyl-1,2,4-thiadiazole (compound (1-2)) (5.32 g, 29.72 mmol), N,N-diisopropylethylamine (0.768 g, 5.94 mmol), toluene (3.46 mL), DMF (3.79 mL), and potassium cyanide (2.13 g, 32.7 mmol) were mixed, and the reaction mixture was stirred at the same temperature for 4 hr. Thereafter, at 15°C, to the reaction mixture were added toluene (12.16 mL), triethylamine (0.60 g, 5.94 mmol), methanol (1.28 g, 29.72 mmol), and sodium methoxide (0.13 g, 2.43 mmol), and the mixture was further stirred at the same temperature for 1 hr. To the reaction mixture was added water, a partitioning operation was performed twice, and the organic layer was washed with 10% aqueous sodium chloride solution to obtain a toluene solution of the title compound (2-2) in 61% yield (quantitative by NMR).
¹H-NMR (400 MHz, CDCl₃) δ 2.36 (s,3H), 3.47 (s,3H), 9.36 (s,1H);

### [Example 5]

### Production of methyl 3-methyl-1,2,4-thiadiazole-5-carboxylate (3-2)

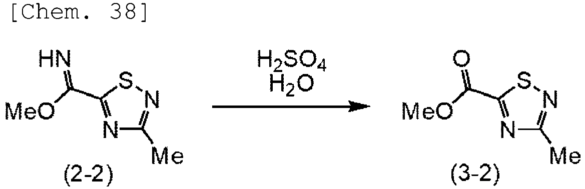

Under a nitrogen atmosphere, a toluene solution of compound (2-2) (concentration 14%) (204.4 g, 0.18 mol) obtained in Example 4 was cooled to 5°C, water and concentrated sulfuric acid (34.7 g (1.9 eq.)) were added dropwise, and the mixture was stirred at 5°C for 1 hr. Thereafter, the reaction mixture was partitioned. To the obtained organic layer was added 10% aqueous sodium carbonate solution until the pH reached 5 to 6, and the mixture was partitioned. The obtained organic layer was quantified, and a toluene solution of the title compound (3-2) was obtained in 96% yield (quantitative by NMR).
¹H-NMR (400 MHz, CDCl₃) δ 2.36 (s,3H), 3.90 (s,3H).

### [Example 6]

### Production of 3-methyl-1,2,4-thiadiazole-5-carbohydrazide (I)

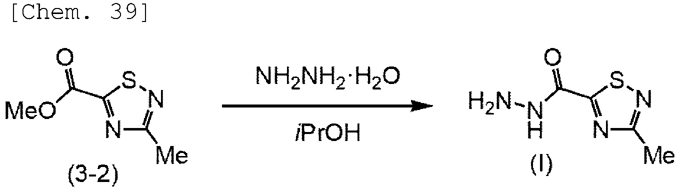

Under a nitrogen atmosphere, hydrazine monohydrate (9.37 g, 0.185 mol) was added to isopropanol (152 mL (5 v/w)), and the mixture was cooled to 0 to 10°C. A toluene solution of compound (3-2) (concentration 15%) (188.4 g, 0.176 mol) obtained in Example 5 was added dropwise thereto, and the mixture was stirred at 0 to 10°C for 12 hr. Thereafter, the reaction mixture was filtered under reduced pressure and the residue was washed with isopropanol (40 mL (4 v/w)) and dried under reduced pressure to obtain the title compound (I) in 92% yield (quantitative by LC).
¹H-NMR (400 MHz, DMSO-d₆) δ 2.56 (s,3H), 4.82 (br,2H), 10.56 (br,1H).

### [Example 7]

### Production of tert-butyl 3-methyl-1,2,4-thiadiazole-5-carboimidate (2-3)

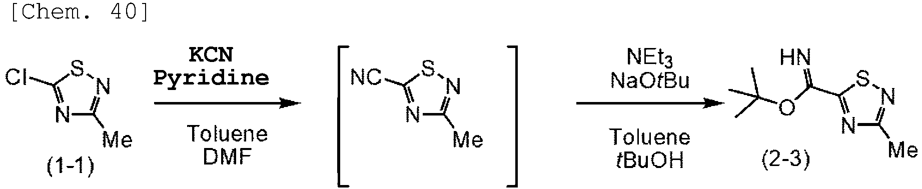

Under a nitrogen atmosphere (normal pressure) at 15°C, compound (1-1) (4 g, 29.72 mmol) obtained in Reference Example 1, pyridine (0.470 g, 5.94 mmol), toluene (3.46 mL), DMF (3.79 mL), and potassium cyanide (2.13 g, 32.7 mmol) were mixed, and the reaction mixture was stirred at the same temperature for 4 hr. Thereafter, at 15°C, to the reaction mixture were added toluene (12.16 mL), triethylamine (0.60 g, 5.94 mmol), tert-butyl alcohol (2.20 g, 29.72 mmol), and sodium tert-butoxide (0.23 g, 2.43 mmol), and the mixture was further stirred at the same temperature for 1 hr. To the reaction mixture was added water, a partitioning operation was performed twice, and the organic layer was washed with 10% aqueous sodium chloride solution to obtain a toluene solution of the title compound (2-3) in 67% yield (quantitative by NMR).
¹H-NMR (400 MHz, CDCl₃) δ1.38 (s,9H), 2.36 (s,3H), 9.36 (s,1H);

### [Example 8]

### Production of tert-butyl 3-methyl-1,2,4-thiadiazole-5-carboxylate (3-3)

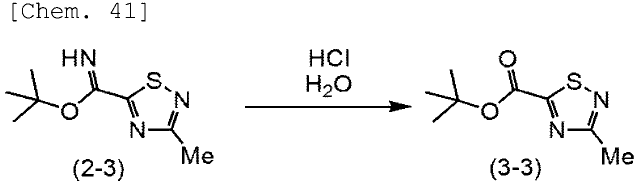

Under a nitrogen atmosphere, a toluene solution of compound (2-3) (concentration 14%) (260.3 g, 0.18 mol) obtained in Example 7 was cooled to 5°C, water and 35% hydrochloric acid (36.0 g (1.9 eq.)) were added dropwise, and the mixture was stirred at 5°C for 1 hr. Thereafter, the reaction mixture was partitioned. To the obtained organic layer was added 10% aqueous sodium carbonate solution until the pH reached 5 to 6, and the mixture was partitioned. The obtained organic layer was quantified and a toluene solution of the title compound (3-3) was obtained in 90% yield (quantitative by NMR).
¹H-NMR (400 MHz, CDCl₃) δ 1.42 (s,9H), 2.36 (s,3H).

### [Example 9]

### Production of 3-methyl-1,2,4-thiadiazole-5-carbohydrazide (I)

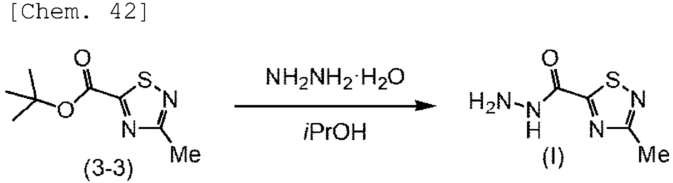

Under a nitrogen atmosphere, hydrazine monohydrate (9.37 g, 0.185 mol) was added to isopropanol (152 mL (5 v/w)), and the mixture was cooled to 0 to 10°C. A toluene solution of compound (3-3) (concentration 15%) (240.0 g, 0.176 mol) obtained in Example 8 was added dropwise thereto, and the mixture was stirred at 0 to 10°C for 12 hr. Thereafter, the reaction mixture was filtered under reduced pressure and the residue was washed with isopropanol (40 mL (4 v/w)) and dried under reduced pressure to obtain the title compound (I) in 93% yield (quantitative by LC).
¹H-NMR (400 MHz, DMSO-d₆) δ 2.56 (s,3H), 4.82 (br,2H), 10.56 (br,1H).

### [Industrial Applicability]

According to the present invention, 3-methyl-1,2,4-thiadiazole-5-carbohydrazide (compound (1)) or a salt thereof, which is an important synthetic intermediate for fezolinetant, can be produced in good yield without using toxic carbon monoxide gas, but by involving 3-methyl-1,2,4-thiadiazole-5-carboimidic acid ester (compound (2)) which is a novel compound. Therefore, a practical production method of fezolinetant can be provided which is safer than conventional methods and can be scaled up.

This application is based on a patent application No. 2023-109328 filed in Japan (filing date: July 3, 2023), the contents of which are incorporated in full herein.

## Claims

1. A method for producing a compound represented by the formula (2): 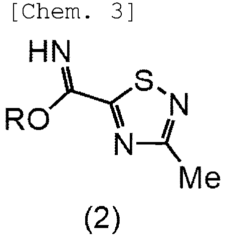
wherein R is a C₁₋₄ alkyl group, or a salt thereof,
comprising a step of reacting a compound represented by the formula (1): 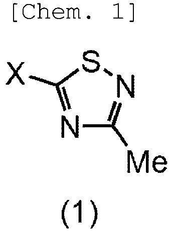
wherein X is a halogen atom, or a salt thereof
with an alkali metal cyanide in a solvent containing an aprotic polar solvent in the presence of an organic base, followed by adding an alcohol represented by the following formula:
[Chem. 2] ROH
wherein R is as defined above, and then reacting the compound with MOR wherein M is an alkali metal and R is as defined above.

2. The production method according to claim 1, wherein
X is a chlorine atom or a bromine atom.

3. The production method according to claim 1, further comprising additional addition of an organic base when adding the alcohol.

4. The production method according to claim 1, wherein
the organic base is triethylamine, N,N-diisopropylethylamine, N-methylmorpholine, pyridine, 2,6-dimethylpyridine, N,N-dimethyl-4-aminopyridine, imidazole, 1,8-diazabicyclo[5.4.0]undec-7-ene, 1,4-diazabicyclo[2.2.2]octane, or 1,5-diazabicyclo[4.3.0]non-5-ene.

5. The production method according to claim 1, wherein
the alkali metal cyanide is potassium cyanide or sodium cyanide.

6. The production method according to claim 1, wherein
the alcohol is methanol or ethanol.

7. The production method according to claim 1, wherein
the aprotic polar solvent is N,N-dimethylformamide, N,N-dimethylacetamide, N-methyl-2-pyrrolidone, or dimethyl sulfoxide.

8. The production method according to claim 1, wherein
the solvent further comprises a non-polar solvent.

9. The production method according to claim 8, wherein
the non-polar solvent is toluene, n-hexane, cyclohexane, or n-heptane.

10. A method for producing a compound represented by the formula (3): 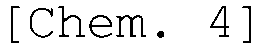 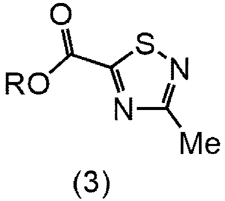 wherein R is as defined above, or a salt thereof,
comprising a step of acid hydrolyzing a compound represented by the formula (2) obtained by the production method according to any one of claims 1 to 9, or a salt thereof.

11. The production method according to claim 10, wherein
the acid is sulfuric acid, hydrochloric acid, hydrobromic acid, nitric acid, phosphoric acid, trifluoroacetic acid, methanesulfonic acid, p-toluenesulfonic acid, aluminum chloride, boron trifluoride, titanium chloride, or zinc chloride.

12. A method for producing a compound represented by the formula (I): 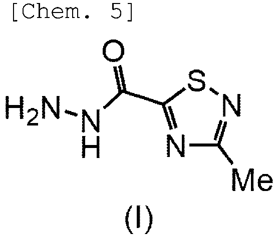 or a salt thereof,
comprising a step of reacting the compound represented by the formula (3) which is obtained by the production method according to claim 10 or a salt thereof with hydrazine or a hydrate thereof in a solvent.

13. A compound represented by the formula (2): 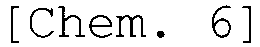 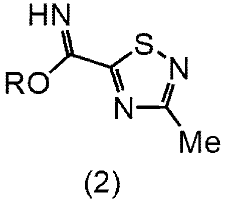 wherein R is a C₁₋₄ alkyl group, or a salt thereof.
